# EUROPÄISCHE PATENTSCHRIFT

(11) **EP 1 735 022 B1**
(45) Veröffentlichungstag und Bekanntmachung des Hinweises auf die Patenterteilung: **08.07.2009**
(21) Anmeldenummer: 05748181.4
(22) Anmeldetag: 15.04.2005
(51) Int. Cl.: A61L 27/26, A61L 27/36

(54) **OSTEOGENES MATRIXKOMPOSIT, VERFAHREN ZU DESSEN HERSTELLUNG SOWIE IMPLANTAT UND SCAFFOLD FÜR DAS TISSUE ENGINEERING MIT EINER BESCHICHTUNG AUS EINEM OSTEOGENEN MATRIXKOMPOSIT**
OSTEOGENIC COMPOSITE MATRIX, METHOD FOR THE PRODUCTION THEREOF AND IMPLANT AND SCAFFOLD FOR TISSUE ENGINEERING PROVIDED WITH A COATING FORMED BY SAID OSTEOGENIC COMPOSITE MATRIX
MATRICE COMPOSITE OSTEOGENE, PROCEDE DE FABRICATION DE LADITE MATRICE ET IMPLANT ET GREFFE SERVANT DE SUPPORT POUR LE GENIE TISSULAIRE, POURVUS D'UN REVETEMENT CONSTITUE DE LADITE MATRICE COMPOSITE OSTEOGENE

(30) Priorität: 15.04.2004 DE 102004018959
(43) Veröffentlichungstag der Anmeldung: 27.12.2006
(73) Patentinhaber: Nexilis AG, 2540 Grenchen (CH)
(72) Erfinder: SCHARNWEBER, Dieter, 01069 Dresden (DE); WORCH, Hartmut, 01181 Dresden (DE); BIERBAUM, Susanne, 01159 Dresden (DE)
(74) Vertreter: Bremi, Tobias Hans
(86) Internationale Anmeldenummer: PCT/DE2005/000728
(87) Internationale Veröffentlichungsnummer: WO 2005/099785

(56) Entgegenhaltungen:
- US-A- 4 544 516
- US-A- 5 116 389
- US-A1- 2003 141 618

## Beschreibung

Die Erfindung betrifft ein Verfahren zur Herstellung eines Implantats oder eines Scaffolds für das Tissue Engineering mit einer Beschichtung aus einem osteogenen Matrixkomposit aus Kollagen und nicht-kollagenen Komponenten der extrazellulären Matrix (ECM-Komponenten), sowie Implantate und Scaffolds für das Tissue Engineering mit einer Beschichtung aus dem osteogenen Matrixkomposit zur Stimulierung und beschleunigten Bildung von Hartgewebe, wie zum Beispiel auf dem Gebiet der Osseointegration von Implantaten in Knochen, sowie eine Beschichtungslösung, welche ein osteogenes Matrixkomposit enthält.

Im Gewebe sind die Zellen in die native extrazelluläre Matrix (ECM) eingebettet, die ein wichtiger Bestandteil der zellulären Umgebung ist. Die native ECM stellt ein hoch geordnetes, gewebespezifisches Netzwerk dar, welches aus Kollagenen, Glykoproteinen, Proteoglykanen und Glykosaminoglykanen (GAG) besteht. Die Zusammensetzung für verschiedene Gewebe und für verschiedene Entwicklungsstadien ist dabei sehr unterschiedlich, so dass die jeweilige Matrix spezifische Eigenschaften hinsichtlich der Wechselwirkung mit Zellen und Wachstumsfaktoren aufweist.

Das hauptsächliche Strukturprotein der nativen Knochenmatrix ist Kollagen Typ I, aber verschiedene andere Matrixproteine wie Proteoglykane und Glykoproteine können mit dem Kollagen wechselwirken und Struktur und Funktion der Matrix beeinflussen. Diese nicht-kollagenen ECM Proteine erfüllen spezifische Funktionen in der Matrix. So weist Fibronektin neben zellbindenden auch kollagen- und GAG-bindende Eigenschaften auf [Stamatoglou und Keller, 1984, Biochim Biophys Acta. Oct 28; während small leucin rich proteins (SLRP's) wie Decorin nicht nur in der Organisation der nativen ECM eine Rolle spielen (Decorin moduliert die Fibrillenbildung *in vivo*), sondern auch Wachstumsfaktoren wie TGF-β oder selbst eine Rolle als Signalmoleküle spielen [Kresse und Schönherr, 2001, J Cell Phys 189:266-274].

Proteoglykane und Glykoproteine unterscheiden sich durch ihren Glykosilierungsgrad, wobei der Zuckeranteil der besonders hoch glykosilierten Proteoglykane aus verschiedenen Glykosaminoglykanen besteht. Die Verteilung dieser Ketten kann, wie beispielsweise für das Decorin, gewebespezifisch sein (Chondroitinsulfat im Knochen, Dermatansulfat in der Haut). Die Glykosaminoglykane sind grosse, unverzweigte Polysaccharide, die aus sich wiederholenden Disacchariden bestehen, welche sich beispielsweise aus N-Acetylgalactosamin, N-Acetylglucosamin, Glucuronat oder Iduronat zusammensetzen, die zu unterschiedlichen Graden sulfatiert sind. Die Zuckerketten liegen *in vivo* gebunden an die Proteoglykane vor und spielen eine wichtige Rolle in der Funktion dieser Proteine, i.e. in der Wachstumsfaktorbindung und -modulation [Bernfield et al, 1999, Annu Rev Biochem, 68:729-777].

Einzelne ECM-Bestandteile, insbesondere Kollagen, werden bereits für die biokompatible Modifikation von Scaffolds und Implantaten genutzt, um die Zelladhäsion und das Einheilverhalten zu verbessern. Die US 2003/141618 A1 beschreibt ein Verfahren zur Herstellung einer Orientierungsschicht aus Polymer-Material. Dabei kann durch Fibrillogenese unter Scherfluss eine ein- oder mehrschichtige Struktur aus orientierten Kollagenfasern erreicht werden, welche als solche für therapeutische Zwecke anwendbar ist. Neben Kollagen werden in verschiedenen Anwendungen weitere ECM-Komponenten wie Polysaccharide genutzt. So wurde Knochengewebe mit Glykosaminoglykanen vernetz, um einen drei-dimensionalen Scaffold für Anwendungen in der Gewebekultur herzustellen (WO01/02030A2).

Eine Chondroitinsulfat enthaltende Mischung wird für die Reparatur von Knochendefekten verwendet; diese fördert die Heilung des Bindegewebes, hauptsächlich aufgrund des Anteils an Aminozuckern und einer dadurch bedingten gesteigerten Matrixproduktion (WO 98/27988, WO 99/39757). In Kombination mit Kollagen werden pflanzliche Polysaccharide als Wundauflagen verwendet (EP 0140569 A2), und eine Kombination von Chitosan und GAG's wird als Agens für die Stimulierung der Regeneration von Hartgewebe beschrieben (WO 96/02259). Kollagen-GAG-Gemische werden dabei durch Säure-Kopräzipitation hergestellt, wobei ein unstrukturiertes Präzipitat und keine definierten Kollagenfibrillen vergleichbar denen in der nativen ECM entstehen (US 4448718, US 5716411, US 6340369).

Mit fortschreitender Verfügbarkeit rekombinanter Wachstumsfaktoren sind solche osteoinduktiven Faktoren, die die Wechselwirkungen zwischen Implantat und umgebendem Gewebe aktiv beeinflussen, zunehmend von Interesse für Implantatanwendungen [Anselme K (2000). Biomaterials 21,667-68]. Im Zusammenhang mit der Knochenheilung sind insbesondere die ,bone morphogenetic proteins' (BMP 2, 4-7) interessant, da sie die Differenzierung mesenchymaler Stammzellen in Chondrozyten und Osteoblasten und die Neubildung von Knochen induzieren [Celeste AJ, Taylor R, Yamaji N, Wang J, Ross J, Wozney JM (1994) J. Cell Biochem. 16F, 100; Wozney JM, Rosen V (1993) Bone morphogenetic proteins in Mundy, GR, Martin TJ (Ed.) Physiology and pharmacology of bone. Handbook of experimental pharmacology, vol. 107. Springer Verlag, Berlin, 725-748].

Aufgrund dieser starken knocheninduzierenden Effekte werden rekombinante BMPs in verschiedenen Carriermaterialien eingesetzt, um die Regeneration von Knochen zu fördern und zu verbessern. Effektive Träger für morphogenetische Proteine sollen diese binden, vor Hydrolyse schützen, eine nachfolgende, kontrollierte Freisetzung ermöglichen und die assoziierten Zellreaktionen fördern. Darüber hinaus sollen solche Träger biokompatibel und biodegradabel sein. Bevorzugte Trägermaterialien für BMPs sind beispielsweise xenogene Knochenmatrix (WO 99/39757) oder natürliches Gewebe nachträglich vernetzt mit GAG's (WO 01/02030 A2), oder HAP, Kollagen, TCP, Methylcellulose, PLA, PGA, und verschiedene Kopolymere (EP 0309241 A2, DE 19890329, EP 0309241 A2, DE 19890906, WO 8904646 A1, DE 19890601). Weitere Anwendungen beinhalten ein quervernetztes synthetischen Polymer, welches zusätzliche Komponenten wie GAG's, Kollagen oder bioaktive Faktoren enthalten kann (WO 97/22371), oder quervernetztes Kollagen gemischt mit Glykosaminoglykanen und osteogenen Faktoren (WO 91/18558, WO 97/21447). Das Kollagen-GAG-Gemisch wird dabei ebenfalls durch Säure-Kopräzipitation hergestellt.

Die Verwendung rekombinanter Wachstumsfaktoren ist mit grossen Nachteilen verbunden. Da die rekombinanten Faktoren meist eine niedrigere Aktivität aufweisen als die endogenen, natürlich im Gewebe vorkommenden Faktoren, sind, um einen Effekt *in vivo* zu erzielen, unphysiologisch hohe Dosen notwendig. Die Gabe von rekombinanten Faktoren kann nur sehr unvollständig die Wirkung von endogenen Faktoren simulieren.

Durch die Verwendung von Faktoren, welche die Wirkung der BMPs (Bone morphogenetic protein) fördern, oder durch den Einsatz von Zellen, die die Wachstumsfaktoren an Ort und Stelle exprimieren können, wird versucht, dieses Problem zu minimieren oder zu umgehen (WO 97/21447, WO 98/25460). Weitere Probleme können durch die Tatsache entstehen, dass Rezeptoren für BMP in vielen verschiedenen Geweben auftreten, die Funktion dieser Wachstumsfaktoren also nicht auf den Knochen limitiert ist.

Aufgabe der vorliegenden Erfindung ist es, ein biokompatibles und biodegradables Matrixkomposit anzugeben, welches die Knochenanlagerung und das Knochenwachstum in der unmittelbaren Umgebung und auf der Oberfläche von mit dem Matrixkomposit beschichteten Implantaten fördert und beschleunigt, und welches insbesondere für die Beschichtung von synthetischen, metallischen oder keramischen Implantaten verwendbar ist. Ein weiteres Ziel der Erfindung ist eine Beschichtung von Trägermaterialien (Scaffolds) für das Tissue Engineering, welche die Erzeugung von Hartgewebe in vitro und folgend *in vivo* unterstützt.

Die Erfindung beruht auf der wissenschaftlichen Erkenntnis, dass für Implantate im Knochenkontakt in den allermeisten Fällen aufgrund des umgebenden Gewebes und der Durchblutung eine ausreichende Menge an endogenen knochenbildender Faktoren anwesend ist. Auch ist der knocheninduzierende Effekt der BMPs, der unter physiologischen Bedingungen *in vivo* beobachtet werden kann, aller Wahrscheinlichkeit nach nicht durch einen einzelnen Wachstumsfaktortyp ausgelöst, sondern das Resultat der synergistischen Wirkung einer Vielzahl von endogenen Faktoren.

Vor diesem Hintergrund ist eine Implantatbeschichtung erstrebenswert, welche die endogenenen knochenbildenden Faktoren, die an der Implantationsstelle vorhandenen sind, in vorteilhafter Weise ausnützt.

Erfindungsgemäss wird die Aufgabe gelöst durch die Herstellung eines Implantates oder eines Scaffolds für das Tissue Engineering mit einer Beschichtung aus einem osteogenen Matrixkomposit aus Kollagen und mindestens einer nicht-kollagenen ECM-Komponente oder deren Derivaten, bei dem die Kollagenkomponente aus mittels Fibrillogenese erzeugten, unvernetzten Kollagenfibrillen besteht, in die die mindestens eine nicht-kollagene ECM-Komponente oder deren Derivate integriert sind.

Für das osteogene Matrixkomposit werden erfindungsgemäss Bestandteile der extrazellulären Matrix verwendet, die den Matrixbestandteilen, wie sie natürlicherweise im Knochen vorkommen, in Zusammensetzung und Morphologie möglichst ähnlich sind, die biokompatibel und biologisch abbaubar sind, und knochengewebsspezifische Funktionen sowohl in der Bindung und Präsentation von Wachstumsfaktoren haben, als auch direkt die Reaktionen der Zellen beeinflussen können. Dadurch wird den Zellen eine den *in vivo* Gegebenheiten möglichst angenäherte Mikroumgebung geboten, welche die Zellfunktionen und die Reaktion auf knochenbildende Faktoren wie Wachstumsfaktoren positiv beeinflusst.

Der Begriff Kollagen umfasst alle fibrillenbildenden Kollagentypen. Jede Kollagenquelle kommt in Betracht, die nicht-vernetzte, säurelösliche Kollagenmonomere liefert, rekombinant oder aufgereinigt, mit und ohne Telopeptide.

Der Begriff nicht-kollagene ECM-Komponenten umfasst sowohl Glykosaminoglykane als auch nicht-kollagenen Proteine, welche bekannte Bestandteile der nativen ECM sind.

Der Begriff nicht-kollagene Proteine umfasst alle Matrixproteine mit nicht-kollagener (Proteoglykane und Glykoproteine) oder teil-kollagener (FACIT's) Struktur.

Hauptbestandteil des osteogenen Matrixkomposits ist Kollagen vom Typ I, II, III, V, IX, XI, oder deren Kombinationen. Im Prinzip kann jeder fibrillenbildende Kollagentyp verwendet werden, der nicht-vernetzte, säurelösliche Kollagenmonomere liefert, wobei Kollagen I, III und V bevorzugt werden, da diese die im Knochen hauptsächlich vertretenen Kollagene sind.

Als GAG-Komponenten enthält die osteogene Matrixkomposition Chondroitinsulfat A, C, D, E; Dermatansulfat, Keratansulfat, Heparansulfat, Heparin, Hyaluronsäure oder deren Derivate, sowohl einzeln als auch gemischt, wobei Chondroitinsulfat bevorzugt wird. Die verwendeten Zucker sind entweder synthetisch hergestellt oder aus biologischen Quellen isoliert.

Als weitere nicht-kollagene Matrixproteine kann die osteogene Matrixkomposition Fibronektin, Decorin, Biglycan, Laminin oder Versikan, sowohl einzeln als auch gemischt enthalten, wobei Decorin und Biglycan bevorzugt werden. Die verwendeten Proteine sind entweder rekombinant hergestellt oder nativ aus biologischen Quellen isoliert.

Um eine möglichst knochenanaloge Matrix zu generieren, werden bevorzugt Kollagen Typ I, Decorin sowie Biglykan und/oder deren GAG-Ketten wie Chondroitinsulfat eingesetzt. Decorin oder Biglykan werden dabei verwendet, um Bindungen bzw. Synergismen zwischen Matrix, Wachstumsfaktor und Zelle auszunutzen. Eine weitere Möglichkeit, der hier der Vorzug gegeben wird, ist der Einsatz von GAG-Ketten, welche körpereigene Wachstumsfaktoren binden bzw. in ihrer Wirkung potenzieren können; insbesondere das im Knochen häufig vorkommenden Chondroitinsulfat. Durch Kombination von Kollagen mit weiteren GAGs oder Matrixbestandteilen lassen sich auch weitere körpereigene Wachstumsfaktoren für eine beschleunigte Einheilung utilisieren, wie beispielsweise VEGF durch Heparansulfat zur Förderung der Invaskularisierung.

Erfindungsgemäß wird ein osteogenes Matrixkomposit aus Kollagen und mindestens einer nicht-kollagenen ECM-Komponenten oder deren Derivaten so hergestellt, dass Kollagenfibrillen mittels Fibrillogenese erzeugt werden, dass vor der Fibrillogenese mindestens eine nicht-kollagene ECM-Komponente oder deren Derivate zugegeben wird. Die so erzeugten Kollagenfibrillen können nach der Resuspension in Wasser oder in einem Puffersystem als Beschichtungslösung genutzt oder lyophylisiert werden.

Die Fibrillogenese (d. h. die Ausbildung von Kollagenfibrillen) läuft unter folgenden Bedingungen ab: Temperaturbereich von 4°C bis 40°C, vorzugsweise 25 °C bis 37 °C, Kollagenkonzentration 50 bis 5000 µg/ml, vorzugsweise 250 bis 1000 µg/ml, pH 4 bis pH 9, vorzugsweise pH 6 bis pH 8, Phosphatgehalt bis 500 mMol/l, vorzugsweise 30 bis 60 mMol/l, NaCl-Gehalt bis zu 1000 mMol/l, vorzugsweise bis zu 300 mMol/l.

Durch das erfindungsgemäße Herstellungsverfahren entsteht ein osteogenes Matrixkomposit mit einer definierten Struktur und Zusammensetzung vergleichbar der Situation in der nativen ECM.

Charakteristisch für Kollagenfibrillen *in vivo* ist eine geordnet gegeneinander versetzte laterale Assoziation der Kollagenmonomere, wobei ein typisches Bandenmuster mit einer Periodizität von 64 bis 67 nm entsteht. Diese Assoziation ist unter anderem durch das Ladungsgmuster der Monomere bedingt. Fibrillenbildung *in vitro* wird dadurch ausgelöst, dass der pH, die Temperatur und die Ionenstärke einer kalten, sauren Kollagenlösung auf Werte in der Nähe der physiologischen Parameter gebracht werden.

Glykosaminoglykane oder andere Matrixkomponenten werden vor der Fibrillogenese zu der Kollagenmonomere enthaltenden Lösung hinzugefügt und dadurch in den folgenden Prozess der Fibrillogenese einbezogen. Durch die Anwesenheit der nicht-kollagenen ECM-Komponenten während der Fibrillogenese werden diese in die entstehende Fibrille integriert und es wird eine Matrix gebildet, die bezüglich der verwendeten Komponenten der Zusammensetzung und Struktur der nativen ECM entspricht.

Während der Fibrillogenese *in vitro* bildet Kollagen die charakteristischen quergestreiften Fibrillen analog den *in vivo* Strukturen, wobei die Struktur der entstehenden Fibrillen durch die Prozessparameter (pH, Ionenstärke, Phosphatkonzentration) und durch Art und Menge der in der Reaktionslösung vorliegnden nicht-kollagenen Komponenten beeinflusst wird. Für *in vivo* matrix-modifizierende Proteoglykane wie Decorin erhält man auf diese Weise die größtmögliche Annährung an ihre native biologische Funktion, da sie so auch unter *in vitro* Bedingungen die Struktur der entstehenden Fibrillen beeinflussen können.

Im Gegensatz zur Strukturbildung infolge Aggregation durch Fibrillogenese kann im sauren Milieu Kollagenaggregation auch durch die Zugabe eines Polyanions, wie es die Glykosaminoglykane darstellen, ausgelöst werden, wobei die zwischen dem GAG und dem Kollagenmonomer existierenden elektrostatischen Wechselwirkungen ursächlich sind. In einem solchen Säurepräzipitat kann die Assoziation der Kollagenmonomere nicht mit derjenigen unter angenähert physiologischen Bedingungen verglichen werden. Es bildet sich entweder ein amorphes Präzipitat oder, bei entsprechenden Mengenverhältnissen und hinreichender Übereinstimmung der Ladungsmuster, ein polymorphes Aggregat wie segmentlong-spacing Kristallite.

Für Glykoproteine oder Proteoglykane wie Decorin besteht keine Möglichkeit einer Präzipitation aus dem sauren Milieu.

Um möglichst dicht an den Gegebenheiten *in vivo* zu bleiben, werden die Kollagenfibrillen erfindungsgemäß nicht vernetzt. Eine Vernetzung würde zwar die Stabilität erhöhen, sich aber andererseits nachteilig auf solche Domänen auswirken, die spezifische Bindungen mit endogenen knochenbildenden Faktoren eingehen können. Insbesondere für die Funktion der GAG's ist dies von Bedeutung, da ihre wachstumsfaktorbindenden Eigenschaften auf einer freien Beweglichkeit der Zuckerkette beruhen, die durch die Vernetzung eingeschränkt wird. Gleichzeitig können die Zucker so aus der Matrix freigesetzt werden, was für die Präsentation der Wachstumsfaktoren an der Zelloberfläche von Bedeutung ist.

Die Erfindung umfasst die Verwendung des erfindungsgemäßen osteogenen Matrixkomposits für die Beschichtung von Implantaten oder Scaffolds für das Tissue Engineering.

Unter Implantat im Sinne der Erfindung werden alle metallischen, keramischen und polymeren oder aus verschiedenen Materialgruppen zusammengesetzten Implantate verstanden, deren Oberflächen zumindest teilweise in Kontakt zu Knochengewebe stehen. Ebenso alle metallischen, keramischen und polymeren oder aus verschiedenen Materialgruppen zusammengesetzten Strukturen, die als Scaffold für das Tissue Engineering von Hartgewebe dienen.

Das zuvor beschriebene osteogene Matrixkomposit eignet sich insbesondere für die Beschichtung von nicht degradierbaren Implantaten im Knochenkontakt, wie künstliche Hüftgelenke, Zahnimplantate oder andere lasttragende Applikationen, für die eine schnelle und feste Integration des Implantates in den Knochen notwendig ist.

Die osteogene Matrix in Kombination mit einem dreidimensionalen, degradierbaren Implantat, das als Knochenersatz implantiert wird, kann vorteilhaft die Integration und den Umbau des Implantats sowie die Knochenneubildung beschleunigen. Diese Implantate können beispielsweise als Grundkomponente partikuläre oder dreidimensionale Strukturen aus Calciumphosphaten, aber auch polymeren Materialien aufweisen.

Für das Tissue Engineering kann die osteogene Matrixkomposition in Kombination mit einem Scaffold vorteilhaft für Proliferation und Differenzierung der knochenbildenden Zellen sein. Als Scaffold kommen alle dreidimensionalen, porösen Strukturen aus synthetischen und/oder natürlichen Polymeren (z.B. Kollagen), Keramik oder Metall einzeln oder in Kombination in Frage, wobei biodegradablen Scaffolds aus Polymer und/oder Keramik der Vorzug gegeben wird.

Durch das osteogene Matrixkomposit werden knochenbildenden Faktoren, wie z. B. Wachstumsfaktoren, die *in vivo* vorliegen, nach der Implantation an die Oberfläche des Implantats gebunden und ihre Aktivität verstärkt. Vorteilhaft werden durch das mit dem osteogenen Matrixkomposit beschichtete Implantat unterschiedliche endogene Faktoren, die an der Implantationsstelle vorhanden sind, rekrutiert.

Zur Herstellung eines Implantats oder eines Scaffolds für das Tissue Engineering wird die das osteogene Matrixkomposit enthaltende Beschichtungslösung genutzt, um das osteogene Matrixkomposit vorteilhaft durch einen dip-coating Prozess auf deren Oberfläche zu immobilisieren. Die Kollagenkonzentration der Beschichtungslösung kann zwischen 0,5 mg/ml bis 5 mg/ml liegen, wobei 1 mg/ml bis 2 mg/ ml der bevorzugte Bereich sind. Die osteogene Matrixkomposit wird durch Inkubation des Implantates für 5 bis 20 Minuten bei Raumtemperatur immobilisiert, anschließend getrocknet und mit Wasser gewaschen. Die Dicke der entstehenden Schicht kann durch die Konzentration der Beschichtungslösung und durch die Anzahl der Prozesswiederholungen beeinflusst werden.

Für die Generierung eines beschichteten dreidimensionalen Scaffolds in Kombination mit dem beschriebenen osteogenen Matrixkomposit wird die Komponentenmischung vorteilhaft vor Beginn der Fibrillenbildung in den Scaffold, der metallischen, keramischen und/oder polymeren Ursprungs sein kann, eingebracht. Die Fibrillogenese wird anschließend durch Temperaturerhöhung ausgelöst. Die *in situ* gebildeten Fibrillen können entweder als Kollagengel verbleiben, oder analog zur Oberflächenbeschichtung getrocknet werden.

Das so hergestellte Implantat oder Scaffold kann vorteilhaft mit den bekannten nichtthermische Verfahren wie Ethylenoxid oder Gamma-Bestrahlung sterilisiert und bei Raumtemperatur gelagert werden.

Das erfindungsgemäß mit einem osteogenen Matrixkomposit beschichtete Implantat oder Scaffolds grenzt sich durch folgende Vorteile von den aus dem Stand der Technik bekannten Lösungen ab:
- Gute biologische Kompatibilität und Funktionalität der erzeugten Matrix durch weitgehend physiologische Zusammensetzung und Struktur aufgrund der Bedingungen in der Herstellung und Einsatz von Komponenten, die denen der natürlichen Zellumgebung entsprechen.
- Hohe Variabilität in Bezug auf einsetzbare Komponenten und deren Anteile in der Komponentenmischung
- Leichte Lagerungs- und Sterilisationsbedingungen
- Hohe Spezifizität und Effizienz durch die Nutzung der körpereigenen osteogenen Faktoren

Anhand der nachfolgenden Ausführungsbeispiele, Vergleichsversuche und Figuren wird die Erfindung näher erläutert.

Dabei zeigen
- **Fig. 1:**: Einfluss von Decorin und Chondroitinsulfat (CS) auf die Bildung von Kollagenfibrillen, gemessen als Zunahme der Trübung einer Fibrillogeneselösung in OD über die Zeit
- **Fig. 2**: AFM-Aufnahmen der Fibrillenstzuktur
- **Fig.3**: In erfindungsgemäßen osteogenen Matrixkompositen vorhandenes Chondroitinsulfat und Decorin
- **Fig. 4**: Bindungsverhalten erfindungsgemäßer osteogener Matrixkomposite für die rekombinanten Wachstumsfaktoren BMP-4 und TGF-1β
- **Fig. 5**: Verhalten von primären Rattencalvaria-Osteoblasten auf verschiedenen erfindungsgemäßen osteogenen Matrixkompositen - Einfluss auf Adhäsion und Osteopontin-Expression
- **Fig.** 6: Aktivität der alkalischen Phosphatase in Ratttencalvarienzellen auf verschiedenen erfindungsgemäßen osteogenen Matrixkompositen nach Zugabe von 4 pmol/cm² BMP-4
- **Fig. 7**: Knochenneubildung an der Implantatoberfläche in Prozent nach 6 Monaten im Minischwein-Kiefer

### Ausführungsbeispiel 1

### Fibrillenstruktur nach Fibrillogenese unter verschiedenen Bedingungen

Für die Generierung des osteogenen Matrixkomposits wird eine Lösung von KollagenMonomeren in 0,01 M Essigsäure durch Rühren für 24 Stunden bei 4°C hergestellt. Die Kollagenfibrillen werden anschließend in Anwesenheit der nicht-kollagenen Komponenten durch einen Prozess der Selbstaggregation (Fibrillogenese) in wässrigen Phosphatpufferlösungen bei neutralem pH und einer Temperatur von 37°C gebildet.

Der Bereich für die Bildung der Fibrillen liegt zwischen 0,5 und 5 mg Kollagen/ml und 0,1 bis 5 mg Glykosaminoglykan/ml, wobei 1 mg/ml Kollagen und 0,2 mg/ml GAG und 30 µg/ml Proteoglykan die bevorzugten Bedingungen sind. Die bevorzugten Fibrillogenese-Parameter waren ein 30 mMol/l Phosphatpuffer pH 7.0, entweder mit 135 mMol/l NaCl oder ohne NaCl-Zusatz.

Glykosaminoglykane oder andere Matrixkomponenten werden vor der Fibrillogenese zu den Kollagenmonomeren hinzugefügt und dadurch im folgenden Prozess der Fibrillogenese zumindest teilweise in die entstehenden Fibrillen integriert.

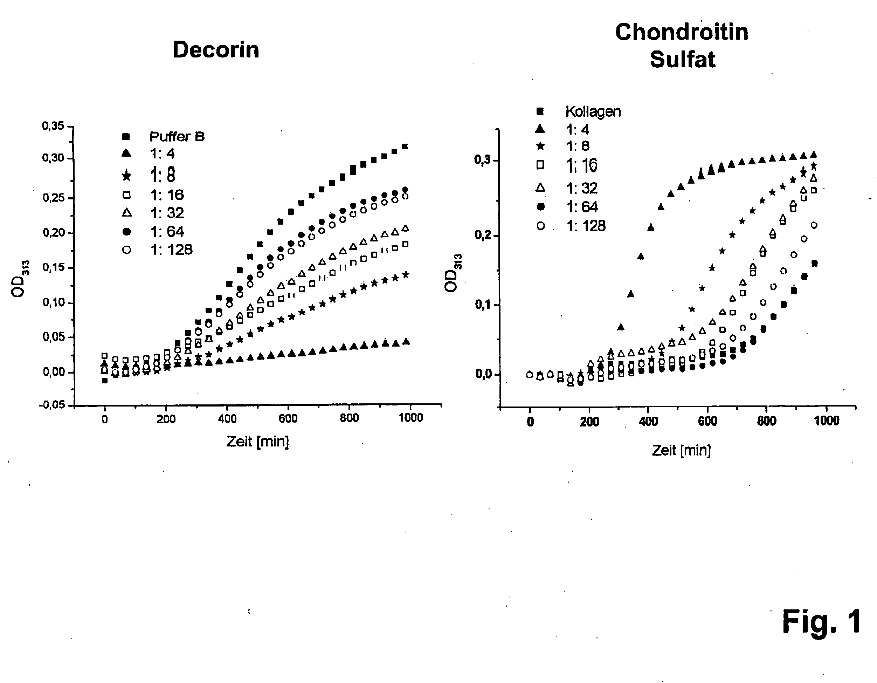

**Fig.1** zeigt in einer Messung der durch die Fibrillenbildung bewirkten Trübung einer Lösung, über die Zeit, dass steigende Mengen von Decorin (angegeben in molaren Verhältnissen) eine Verlangsamung der Bildungskinetik und eine Reduktion der maximalen OD-Werte bewirken, indikativ für eine Verringerung des Fibrillendurchmessers. Für Chondroitinsulfat ist ein gegenteiliger Effekt zu beobachten. Bildungsbedingungen: 250 µg/ml Kollagen, 37°C, 30 mMol/l Phosphatpuffer pH 7,4 mit 135 mMol/l NaCl.

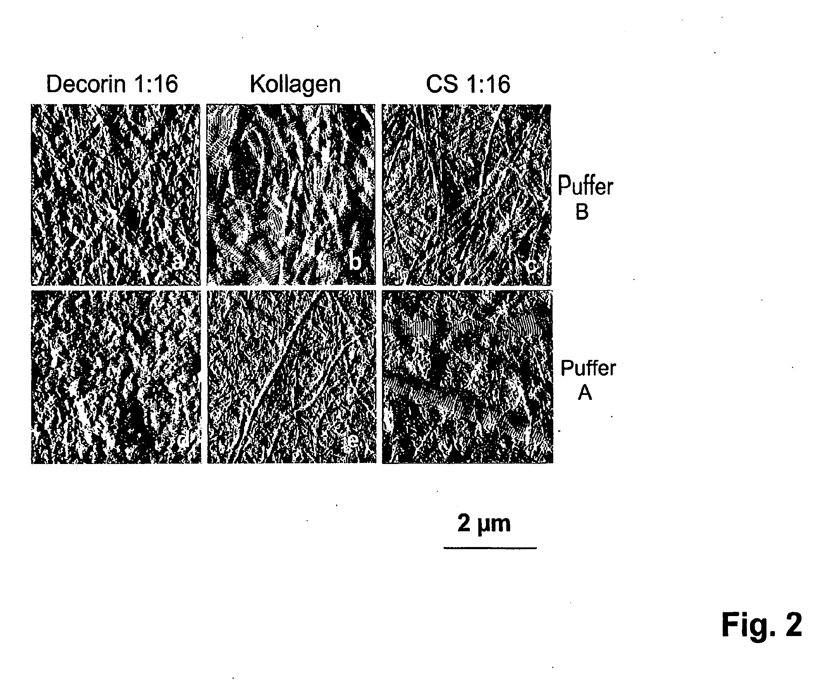

In **Fig. 2** ist der Einfluss der Bildungsbedingungen auf die Struktur der entstehenden Fibrillen in AFM-Aufnahmen dokumentiert. Zugabe von Decorin verringert unter allen Bedingungen den Fibrillendurchmesser (a und d). Für Chondroitinsulfat ist insbesondere unter Bedingungen niedriger Ionenstärke eine deutlich heterogenere Verteilung der Fibrillendurchmesser sichtbar unter Zunahme des durchschnittlichen Fibrillendurchmessers (f), während der Effekt bei höheren Ionenstärken nicht offensichtlich ist (c). b und e zeigen die Fibrillenstruktur ohne nicht-kollagene Zusätze. Bildungsbedingungen: 250 µg/ml Kollagen, 37°C, 30 mMol/l Phosphatpuffer pH 7,4 (Puffer A) oder 30 mMol/l Phosphatpuffer pH 7,4 mit 135 mMol/l NaCl (Puffer B).

In allen Fällen aber bilden die Kollagenmonomere während der Fibrillogenese *in vitro* die charakteristischen quergestreiften Fibrillen analog den *in vivo* Strukturen, wobei die Struktur der entstehenden Fibrillen sowohl durch die Prozessparameter (pH, Ionenstärke, Phosphatkonzentration) als auch durch Art und Menge der zugefügten nicht-kollagenen Komponenten beeinflusst wird. Kollagenfibrillen mit nicht-kollagenen Bestandteilen wie Glykosaminoglykane oder Decorin können demzufolge in einem vergleichsweise breiten Bereich von Massenverhältnissen erzeugt werden, innerhalb derer die Integration des Kollagens in die Fibrillen nicht oder nur geringfügig beeinflusst wird.

### Ausführungsbeispiel 2

### Einbau von nicht-kollagenen Komponenten in Kollagenfibrillen

Für die Generierung des osteogenen Matrixkomposits wird eine Lösung von KollagenMonomeren in 0,01 M Essigsäure durch Rühren für 24 Stunden bei 4°C hergestellt. Die Kollagenfibrillen werden anschließend in Anwesenheit der nicht-kollagenen Komponenten durch einen Prozess der Selbstaggregation (Fibrillogenese) in wässrigen Phosphatpufferlösungen bei neutralem pH gebildet. Bildungsbedingungen: 250 µg/ml Kollagen, 37°C, 30 mMol/l Phosphatpuffer pH 7,4 (Puffer A) oder 30 mMol/l Phosphatpuffer pH 7,4 mit 135 mMol/l NaCl (Puffer B) mit unterschiedlichen Chondroitinsulfat- und Decorin-Konzentrationen.

In die Fibrillen integriertes Decorin und Chondroitinsulfat wurde nach waschen und Hydrolysieren der Fibrillen in 500 µl 6 M HCl bei 105°C für 6 Stunden nach der Methode von Pieper et al. [Pieper JS, Hafmans T, Veerkamp JH, van Kuppevelt TH. Development of tailor-made collagen-glycosaminoglycan matrices: EDC/NHS crosslinking, and ultrastructural aspects. Biomaterials 2000;21(6):581-93] bestimmt.

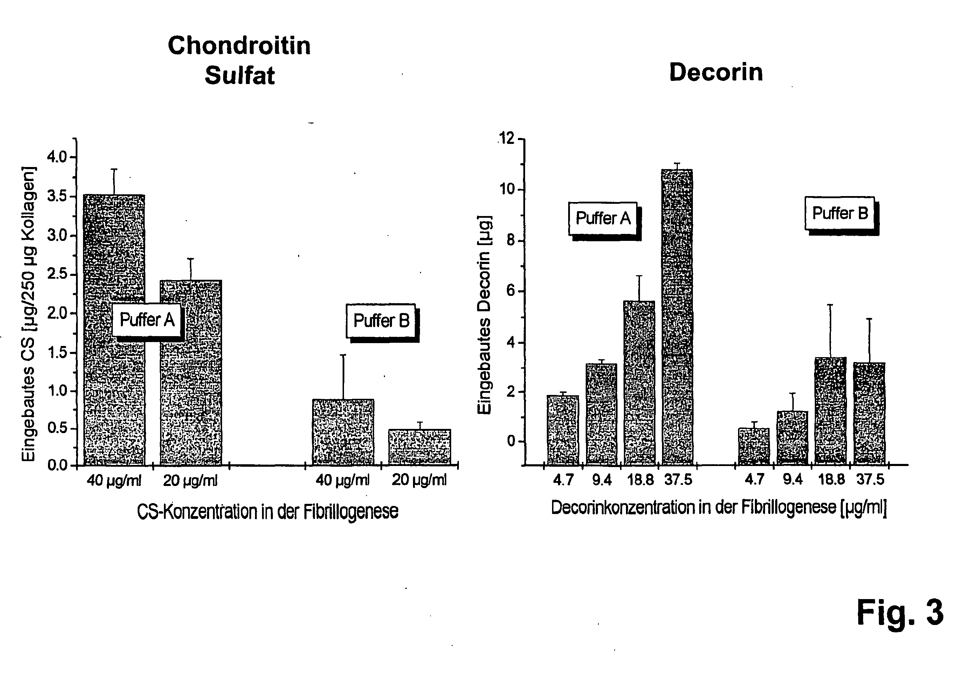

Für Chondroitinsulfat ist das Ausmaß der Integration abhängig von der Ionenstärke des verwendeten Puffersystems. Für geringe Ionenstärken (Puffer A) werden von den eingesetzten 20 µg auf 250 µg Kollagen etwa 2,5 µg CS eingebaut, für hohe Ionenstärken (Puffer B) hingegen nur ein Drittel dieser Menge **(****Fig. 3****)**.

Auch der Einbau von Decorin hängt vom verwendeten Puffersystern ab. Für Puffer A wird ein Drittel der eingesetzten Menge eingebaut, während die Werte für Puffer B wiederum deutlich niedriger waren

### Ausführungsbeispiel 3

### Rekrutierung von Wachstumsfaktoren durch ein mit einem osteogenen Matrixkomposit beschichtetes Implantat

Erfindungsgemäß zusammengesetzte und erzeugte Matrices können ohne den Einsatz rekombinanter Wachstumsfaktoren durch die Rekrutierung körpereigener Wachstumsfaktoren die Knochenbildung und -anlagerung beschleunigen und verbessern. Im Experiment kann ein derartiges Bindungsverhalten nur mit rekombinanten Wachstumsfaktoren nachgewiesen werden.

Eine sandgestrahlte, zylindrische Probe aus TiA16V4 mit einem Durchmesser von 10 mm wird mit Ethanol, Aceton und Wasser gereinigt.

Eine Lösung von 1 mg/ml bovinem Kollagen Typ I in 0.01 M Essigsäure wird durch Rühren über Nacht bei 4°C erzeugt. Zu dieser Lösung werden nicht-kollagene ECM-Komponenten (Glykosaminoglykan 30 µg/ml, Proteoglykane 15 µg/ml) gegeben. Die Mischungen werden auf Eis mit Fibrillogenese-Puffer (60 mMol/l Phosphat, 270 mMol/l NaCl, pH 7,4) versetzt und 18 h bei 37°C inkubiert. Die entstandenen Fibrillen werden abzentrifugiert, gewaschen, homogenisiert und resuspendiert zu einer endgültigen Konzentration von 1 mg/ml.

Die zylindrische Probe wird für 15 min bei RT mit dieser Lösung beschichtet (dip-coating), mit Wasser gewaschen und getrocknet.

Anschließend werden Wachstumsfaktoren (rekombinantes BMP-4 bzw. TGF-1β) durch einen Adsorptionsprozess (4°C, 18h, aus PBS) auf diesen Oberflächen immobilisiert und anschließend mittels ELISA bestimmt.

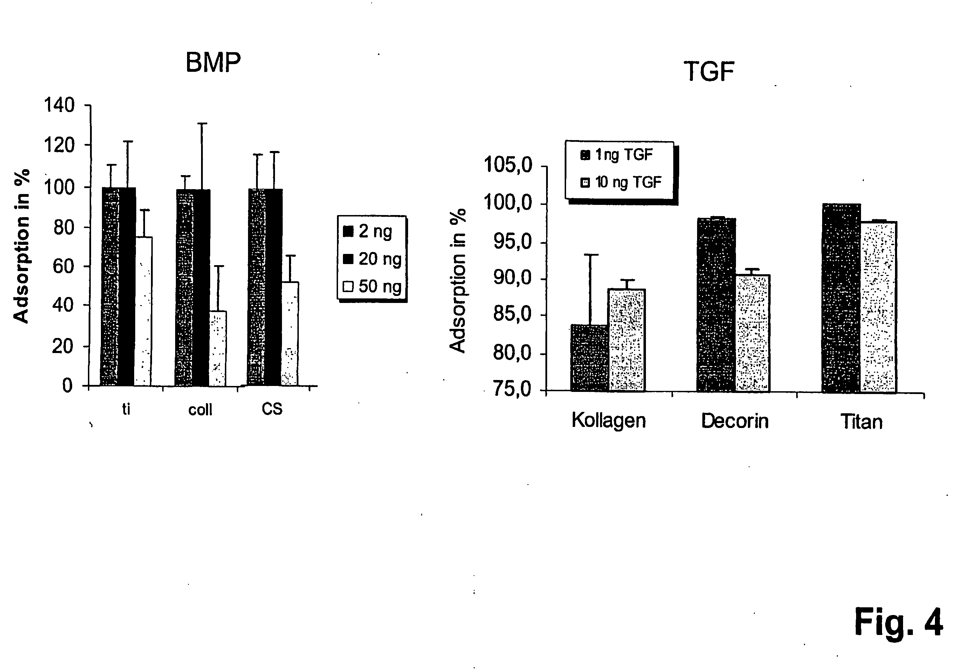

Diese *in vitro* Versuche mit rekombinanten Wachstumsfaktoren zeigen, dass durch den erfindungsgemäßen Zusatz von nicht-kollagenen Komponenten die Bindung der Wachstumsfaktoren rhBMP-4 (insbesondere durch Zusatz von Chondroitinsulfat) oder rhTGF-1β (insbesondere durch Zusatz von Decorin) an die Matrix erhöht wird. Für BMP wird bei geringen Mengen (2 - 20 ng/cm²) kein Effekt beobachtet, bei höheren Mengen (ab 50 ng/cm²) erfolgt aber eine um etwa 10% höher Bindung an die chondroitinsulfathaltige gegenüber der reinen Kollagenschicht, dargestellt in % der eingesetzten Menge **(****Fig. 4****).**

Für rhTGF-1β ist sowohl für 1 ng/cm² als auch für 10 ng/cm² auf decorinhaltigen Oberflächen eine erhöhte Bindung feststellbar.
Bildungsbedingugen der Matrix: 500 µg/ml Kollagen, 30 µg/ml Decorin bzw. Chondroitinsulfat, 37°C, 30 mMol/l Phosphatpuffer pH 7,4 mit 135 mMol/l NaCl.

### Ausführungsbeispiel 4

### Untersuchungen mit Rattencalvarien-Osteoblasten auf verschiedenen Matrixkompositen

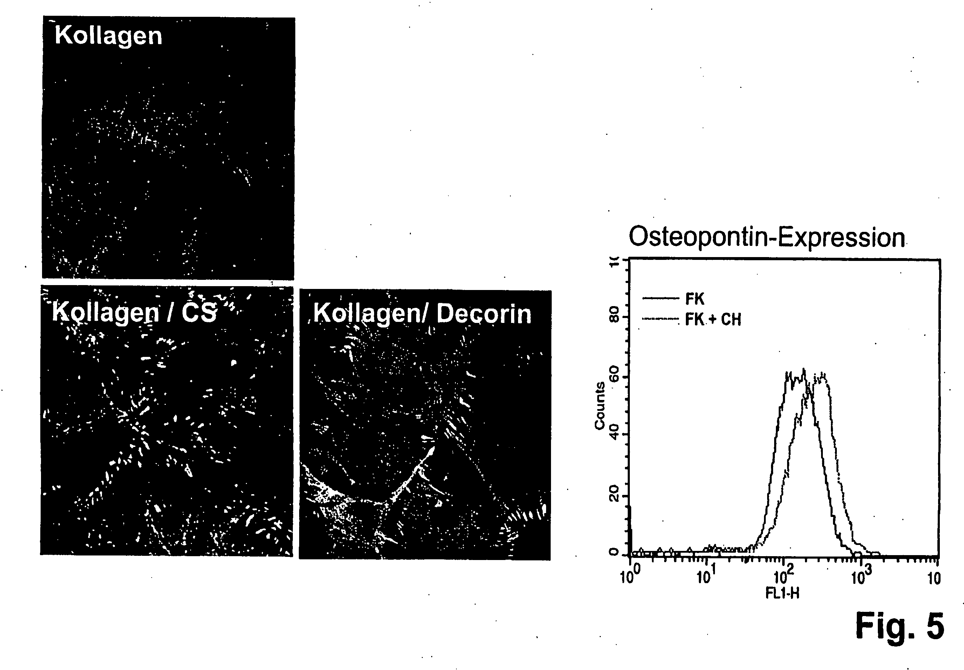

**Fig. 5** zeigt das Verhalten von primären Rattencalvarien-Osteoblasten auf verschiedenen Matrices. Initiale Adhäsion der Zellen auf unterschiedlichen Matrixkompositionen wurde über Zellmorphologie, zytoskeletale Organisation (Aktin-Färbung mit Phalloidin) und Bildung der Focal Adhesion Complexes mittels Integrin-Rezeptoren (Immunofärbung gegen Vinculin) analysiert. Adhäsion war nach 2 Stunden am ausgeprägtesten auf Kollagen-CS-Matrices gefolgt con Kollagen-Decorin. Auch die Bildung der FACS (grün-gelbe Punkte und rot an den Enden der Aktinfibrillen) wurde von Decorin und besonders CS befördert und beschleunigt. Kontrollen mit reinen Kollagen-Matrices zeigten wesentlich weniger FACS nach 2 Stunden.
Der Einfluss der Matrixkomposition auf die Differenzierung der Osteoblasten wurde über die Expression des Markerproteins Osteopontin mittels fluoreszenzaktiviertem Cell Scanning untersucht. Osteoblasten auf Kollagen-CS-Oberflächen produzieren nach 8 Tagen 5 mal mehr Osteopontin (≈ 2500 Fluoreszenzeinheiten) als Zellen auf reine Kollagenoberflächen (≈ 500 Fluoreszenzeinheiten). Bildungsbedingungen der Matrix: 500 µg/ml Kollagen, 30 µg/ml Decorin bzw. Chondroitinsulfat, 37°C, 30 mMol/l Phosphatpuffer pH 7,4 mit 135 mMol/l NaCl.

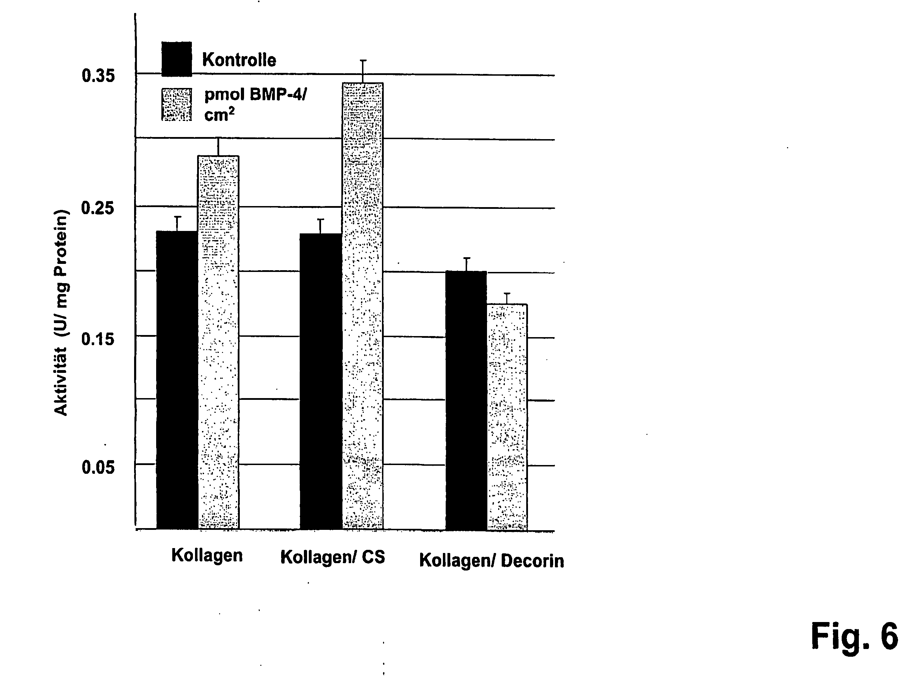

Weitere Untersuchungen mit Rattencalvarien-Osteoblasten zeigen unterschiedliche Zelleaktionen auf rhBMP-4. in Abhängigkeit von der Zusammensetzung der Trägermatrix. **Fig. 6** zeigt die Aktivität der alkalischen Phosphatase in Aktivitätseinheiten U pro mg Protein nach Zugabe von 4 pmol/ cm² rhBMP-4 zu Rattencalvarienzellen. Auf decorinhaltigen Matrices wird die BMP-Aktivität heruntergeregelt, während sie auf chondroitinsulfathaltigen Matrices verstärkt wird. Bildungsbedingungen der Matrix: 500 µg/ml Kollagen, 30 µg/ml Decorin bzw. Chondroitinsulfat, 37°C, 30 mMol/l Phosphatpuffer pH 7,4 mit 135 mMol/l NaCl.

### Ausführungsbeßspiel 5

### Tierversuche

In Tierversuchen wurde überraschend festgestellt, dass mit rekombinanten Wachstumsfaktoren versehene Matrices bezüglich der induzierten Knochenbildung deutlich schlechter abschneiden als die erfindungsgemäßen nicht vernetzten osteogenen Matrixkomposite auf der Basis von Kollagen Typ I und Chondroitinsulfat.

Ti-Implantate, welche ringförmige Einschnitte quer zur Achse aufweisen und so ein Defektmodell darstellen, werden mit 1% Triton X-100, Aceton und 96% Ethanol gereinigt, mit destilliertem. Wasser gespült und getrocknet.

Die eingesetzten Implantate werden in zwei aufeinander folgenden Dip-coating Schritten beschichtet mit:
A. Fibrillen aus Kollagen Typ I,
B. Erfindungsgemäßer osteogener Matrixkomposit auf der Basis von Kollagen Typ I und Chrondroitinsulfat nach Ausführungsbeispiel 1
C. Erfindungsgemäßer osteogener Matrixkomposit auf der Basis von Kollagen Typ I und Chrondroitinsulfat nach Ausführungsbeispiel 1

Die Implantate werden, mit destilliertem Wasser gewaschen, luftgetrocknet und für 12 h mit Ethylenoxid bei 42°C sterilisiert. Unmittelbar vor der Implantation wird der Oberflächenzustand C mit rekombinantem BMP-4 (400 ng/ml) bei 4°C über Nacht beschichtet und anschließend getrocknet.

Die Implantate werden in die Unterkiefer von Minischweinen eingesetzt. Der Knochen-Implantat Kontakt wurde nach 6 Monaten histomorphometrisch bestimmt.

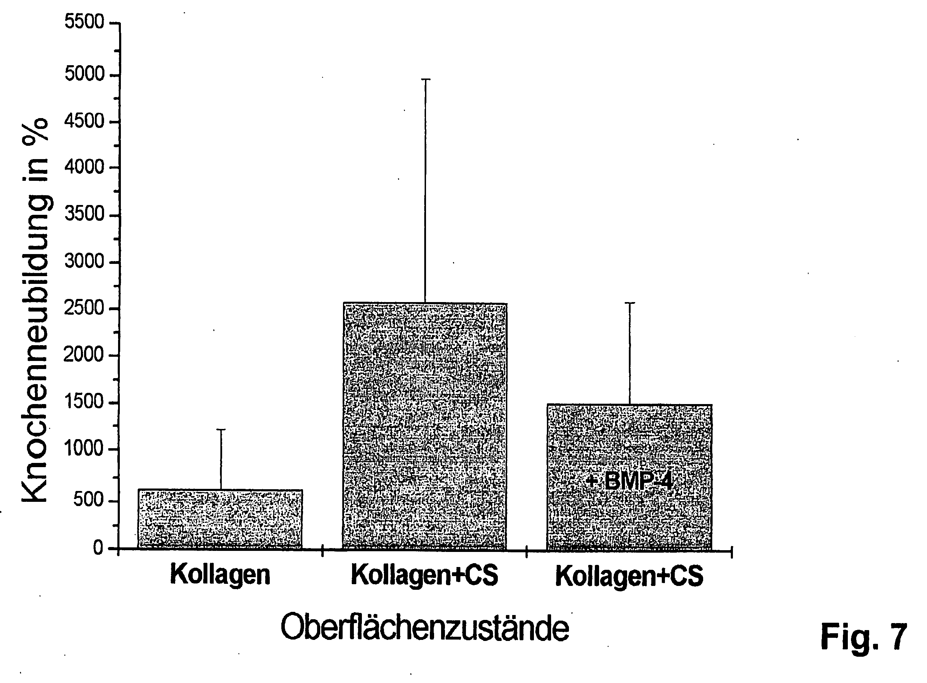

Den höchsten Prozentsatz für diesen Kontakt erhält man für mit der erfindungsgemäße osteogene Matrix auf der Basis von Kollagen und Chondroitinsulfat beschichtete Implantate (27,8%), während Implantate mit derselben Beschichtung und rekombinantern BMP-4 und der Kombination um 15% und damit deutlich niedriger liegen. Die geringsten Werte erhält man für die reine Kollagenbeschichtung (12,8%) **(****Fig. 7****)**.

In der Erfindungsbeschreibung werden folgende Abkürzungen verwendet:
- bFGF: Basic fibroblastic growth factor
- BMP: Bone morphogenetic protein
- ECM: Extrazelluläre Matrix
- EGF: Endothelial growth factor
- FACITs: Fibril associated collagen with interrupted triple helix
- FACS: Focal adhesion contacts
- FGF: Fibroblastic growth factor
- GAG: Glykosaminoglykan
- HAP: Hydroxylapatit
- IGF-I: Insuline-like growth factor
- PGA: Polyglygolic acid
- PLA: Polylactic acid
- SLRP: Small leucine rich protein
- TCP: Tricalziuimphosphat Phasen
- TES: (N-[Tris(hydroxymethyl)methyl]-2-aminoethane-sulfonic acid)-
- TGF-β: Transforming growth factor β
- VEGF: Vascular endothelial growth factor
- WF: Wachstumsfaktor

## Patentansprüche

1. Verfahren zur Herstellung eines Implantates oder eines Scaffolds für das Tissue Engineering mit einer Beschichtung aus einem osteogenen Matrixkomposit **dadurch gekennzeichnet, dass** es sich bei dem osteogenen Matrixkomposit um ein Matrixkomposit aus Kollagen und mindestens einer nicht-kollagenen ECM-Komponente oder deren Derivaten handelt, wobei die Kollagenkomponente aus mittels Fibrillogenese erzeugten, unvernetzten Kollagenfibrillen besteht und wobei in diese die mindestens eine nicht-kollagene ECM-Komponente oder deren Derivate integriert sind, wobei vor der Fibrillogenese die mindestens eine nicht-kollagene ECM-Komponente oder deren Derivate zugegeben werden, und dass entweder die so erzeugten Kollagenfibrillen in Wasser oder in einem Puffer resuspendiert werden und anschliessend auf der Oberfläche des Implantates oder des Scaffolds in einem dip-coating Prozess immobilisiert werden, oder dass bei Herstellung eines Scaffolds für das Tissue Engineering die Fibrillenbildung in dem Scaffold ausgelöst wird, wo die in situ entstandenen Fibrillen entweder als Gel verbleiben oder getrocknet werden.

2. Verfahren zur Herstellung eines Implantates oder eines Scaffolds nach Anspruch 1, **dadurch gekennzeichnet, dass** die nicht-kollagenen ECM-Komponenten Glykosaminoglykane enthalten.

3. Verfahren zur Herstellung eines Implantates oder eines Scaffolds nach Anspruch 1 oder 2, **dadurch gekennzeichnet, dass** die nicht-kollagene ECM-Komponente Chondroitinsulfat vom Typ A, C, D, oder E, Dermatansulfat, Keratansulfat, Heparansulfat, Heparin, Hyaluronsäure und deren Derivate, einzeln oder gemischt enthält.

4. Verfahren zur Herstellung eines Implantates oder eines Scaffolds nach einem der Ansprüche 1-3, **dadurch gekennzeichnet, dass** die nicht-kollagene ECM-Komponente nicht-kollagene Matrixproteine enthält.

5. Verfahren zur Herstellung eines Implantates oder eines Scaffolds nach einem der Ansprüche 1-4, **dadurch gekennzeichnet, dass** die nicht-kollagene ECM-Komponente als nicht-kollagene Matrixproteine Fibronektin, Decorin, Biglycan, Laminin, Versikan einzeln oder gemischt enthält.

6. Verfahren zur Herstellung eines Implantates oder eines Scaffolds nach einem der Ansprüche 1-5, **dadurch gekennzeichnet, dass** die Kollagenkomponente aus einem der Kollagene I, II, III, V, IX, XI, oder deren Kombinationen besteht.

7. Verfahren zur Herstellung eines Implantates oder eines Scaffolds nach einem der Ansprüche 1-6, **dadurch gekennzeichnet, dass** die Fibrillogenese unter den Bedingungen, Temperaturbereich von 4°C bis 40°C, vorzugsweise 25°C bis 37°C, Kollagenkonzentration 50 bis 5000 µg/ml, vorzugsweise 250 bis 1000 µg/ml, pH 4 bis pH 9, vorzugsweise pH 6 bis pH 8, Phosphatgehalt bis 500 mMol/l, vorzugsweise 30 bis 60 mMol/l, NaCl-Gehalt bis zu 1000 mMol/l, vorzugsweise bis zu 300 mMol/l durchgeführt wird.

8. Implantat oder Scaffold für das Tissue Engineering mit einer Beschichtung aus einem osteogenen Matrixkomposit, insbesondere hergestellt nach einem Verfahren nach einem der vorhergehenden Ansprüche, **dadurch gekennzeichnet, dass** es sich bei dem osteogenen Matrixkomposit um ein Matrixkomposit aus Kollagen und mindestens einer nicht-kollagenen ECM-Komponente oder deren Derivaten handelt, wobei die Kollagenkomponente aus mittels Fibrillogenese erzeugten, unvernetzten Kollagenfibrillen besteht und wobei in diese die mindestens eine nicht-kollagene ECM-Komponente oder deren Derivate integriert sind.

9. Implantat oder Scaffold nach Anspruch 8, **dadurch gekennzeichnet, dass** die nicht-kollagenen ECM-Komponenten Glykosaminoglykane enthalten.

10. Implantat oder Scaffold nach Anspruch 8 oder 9, **dadurch gekennzeichnet, dass** die nicht-kollagene ECM-Komponente Chondroitinsulfat vom Typ A, C, D, oder E, Dermatansulfat, Keratansulfat, Heparansulfat, Heparin, Hyaluronsäure und deren Derivate, einzeln oder gemischt enthält.

11. Implantat oder Scaffold nach einem der Ansprüche 8-10, **dadurch gekennzeichnet, dass** die nicht-kollagene ECM-Komponente nicht-kollagene Matrixproteine enthält.

12. Implantat oder Scaffold nach einem der Ansprüche 8-11, **dadurch gekennzeichnet, dass** die nicht-kollagene ECM-Komponente als nicht-kollagene Matrixproteine Fibronektin, Decorin, Biglycan, Laminin, Versikan einzeln oder gemischt enthält.

13. Implantat oder Scaffold nach einem der Ansprüche 8-12, **dadurch gekennzeichnet, dass** die Kollagenkomponente aus einem der Kollagene I, II, III, V, IX, XI, oder deren Kombinationen besteht.

14. Beschichtungslösung, enthaltend ein osteogenes Matrixkomposit aus Kollagen und mindestens einer nicht-kollagenen ECM-Komponente oder deren Derivaten, **dadurch gekennzeichnet, dass** die Kollagenkomponente aus mittels Fibrillogenese erzeugten, unvernetzten Kollagenfibrillen besteht und dass in diese die mindestens eine nicht-kollagene ECM-Komponente oder deren Derivate integriert sind.

## Claims

1. A method for the production of an implant or of a scaffold for tissue engineering having a coating of an osteogenic matrix composite, **characterized in that** the osteogenic matrix composite is a matrix composite of collagen and at least one non-collagenic ECM-component or its derivatives, wherein the collagen component consists of non-crosslinked collagen fibrils produced by means of fibrillogenesis, wherein at least one non-collagenic ECM-component or its derivatives are integrated into said fibrils, wherein before fibrillogenesis the at least one non-collagenic ECM-component or its derivatives are added, and **in that** the collagen fibrils produced in this way are resuspended in water or in a buffer and subsequently immobilized on the surface of the implant or of the scaffold in a dip-coating process, or that during the production of a scaffold for tissue engineering the fibril formation is induced in the scaffold, where the fibrils formed in situ either remain as a gel or are dried.

2. A method for the production of an implant or of a scaffold according to claim 1, **characterized in that** the non-collagenic ECM-components contain glycosaminoglycans.

3. A method for the production of an implant or of a scaffold according to claim 1 or 2, **characterized in that** the non-collagenic ECM-component contains chondroitin sulphate of type A, C, D, or E, dermatan sulphate, keratan sulphate, heparan sulfate, heparin, hyaluronic acid and their derivatives, individually or mixed.

4. A method for the production of an implant or of a scaffold according to one of claims 1-3, **characterized in that** the non-collagenic ECM-component contains non-collagenic matrix proteins.

5. A method for the production of an implant or of a scaffold according to one of claims 1-4, **characterized in that** the non-collagenic ECM-component contains, as non-collagenic matrix proteins, fibronectin, decorin, biglycan, laminin, versican, individually or mixed.

6. A method for the production of an implant or of a scaffold according to one of claims 1-5, **characterized in that** the collagen component consists of one of the collagens I, II, III, V, IX, XI, or combinations thereof.

7. A method for the production of an implant or of a scaffold according to one of claims 1-6, **characterized in that** the fibrillogenesis is carried out under the conditions, temperature range from 4°C to 40°C, preferably 25°C to 37°C, collagen concentration of 50 to 5000 µg/ml, preferably 250 to 1000 µg/ml, pH 4 to pH 9, preferably pH 6 to pH 8, phosphate content up to 500 mmol/l, preferably 30 to 60 mmol/l, NaCl-content up to 1000 mmol/l, preferably up to 300 mmol/l.

8. An implant or scaffold for tissue engineering having a coating of an osteogenic matrix composite, especially produced by a method according to one of the preceding claims, **characterized in that** the osteogenic matrix composite is a matrix composite of collagen and at least one non-collagenic ECM-component or its derivatives, wherein the collagen component consists of non-crosslinked collagen fibrils produced by means of fibrillogenesis, and wherein at least one non-collagenic ECM-component or its derivatives are integrated into said fibrils.

9. An implant or scaffold according to claim 8, **characterized in that** the non-collagenic ECM-components contain glycosaminoglycans.

10. An implant or scaffold according to claim 8 or 9, **characterized in that** the non-collagenic ECM-component contains chondroitin sulphate of type A, C, D, or E, dermatan sulphate, keratan sulphate, heparan sulfate, heparin, hyaluronic acid and their derivatives, individually or mixed.

11. An implant or scaffold according to one of claims 8-10, **characterized in that** the non-collagenic ECM-component contains non-collagenic matrix proteins.

12. An implant or scaffold according to one of claims 8-11, **characterized in that** the non-collagenic ECM-component contains, as non-collagenic matrix proteins, fibronectin, decorin, biglycan, laminin, versican, individually or mixed.

13. An implant or scaffold according to one of claims 8-12, **characterized in that** the collagen component consists of one of the collagens I, II, III, V, IX, XI, or combinations thereof.

14. A coating solution comprising an osteogenic matrix composite of collagen and at least one non-collagenic ECM-component or its derivatives, **characterized in that** the collagen component consists of non-crosslinked collagen fibrils produced by means of fibrillogenesis, and that at least one non-collagenic ECM-component or its derivatives are integrated into said fibrils.

## Revendications

1. Procédé de préparation d'un implant ou d'un support pour l'ingénierie tissulaire avec un revêtement en un composite de matrice ostéogène, **caractérisé en ce qu'**il s'agit, pour le composite de matrice ostéogène, d'un composite de matrice de collagène et d'au moins un composant de type ECM (matrice extracellulaire) non collagène ou de ses dérivés, le composant de collagène étant constitué par des fibrilles de collagène produites par fibrillogenèse, non réticulées et ledit au moins un composant de type ECM non collagène ou ses dérivés étant intégré(s) dans celles-ci, ledit au moins un composant de type ECM non collagène ou ses dérivés étant ajouté(s) avant la fibrillogenèse et en ce soit que les fibrilles de collagène ainsi obtenues sont remises en suspension dans l'eau ou dans un buffer et ensuite immobilisées à la surface de l'implant ou du support dans un processus de revêtement par immersion soit, lors de la préparation d'un support pour l'ingénierie tissulaire, la formation de fibrilles étant déclenchée dans le support, les fibrilles formées in situ restant sous forme de gel ou étant séchées.

2. Procédé pour la préparation d'un implant ou d'un support selon la revendication 1, **caractérisé en ce que** les composants de type ECM non collagènes contiennent des glycosaminoglycanes.

3. Procédé pour la préparation d'un implant ou d'un support selon la revendication 1 ou 2, **caractérisé en ce que** le composant de type ECM non collagène contient du sulfate de chondroïtine de type A, C, D, ou E, du sulfate de dermatane, du sulfate de kératane, du sulfate d'héparane, de l'héparine, de l'acide hyaluronique et ses dérivés, seuls ou en mélange.

4. Procédé pour la préparation d'un implant ou d'un support selon l'une quelconque des revendications 1-3, **caractérisé en ce que** le composant de type ECM non collagène contient des protéines de matrice non collagènes.

5. Procédé pour la préparation d'un implant ou d'un support selon l'une quelconque des revendications 1-4, **caractérisé en ce que** le composant de type ECM non collagène contient, comme protéines de matrice non collagènes, de la fibronectine, de la décorine, du biglycan, de la laminine, du versican, seuls ou en mélange.

6. Procédé pour la préparation d'un implant ou d'un support selon l'une quelconque des revendications 1-5, **caractérisé en ce que** le composant de collagène est constitué par un des collagènes I, II, III, V, IX, XI, ou leurs combinaisons.

7. Procédé pour la préparation d'un implant ou d'un support selon l'une quelconque des revendications 1-6, **caractérisé en ce que** la fibrillogenèse est réalisée dans les conditions plage de température de 4°C à 40°C, de préférence de 25°C à 37°C, concentration en collagène 50 à 5000 µg/ml, de préférence 250 à 1000 µg/ml , pH 4 à pH 9, de préférence pH 6 à pH 8, teneur en phosphate jusqu'à 500 mmoles/l, de préférence 30 à 60 mmoles/l, teneur en NaCl jusqu'à 1000 mmoles/l, de préférence jusqu'à 300 mmoles/l.

8. Implant ou support pour l'ingénierie tissulaire avec un revêtement en un composite de matrice ostéogène, en particulier préparé selon un procédé selon l'une quelconque des revendications précédentes, **caractérisé en ce qu'**il s'agit, pour le composite de matrice ostéogène, d'un composite de matrice de collagène et d'au moins un composant de type ECM non collagène ou de ses dérivés, le composant de collagène étant constitué par des fibrilles de collagène préparées par fibrillogenèse, non réticulées et ledit au moins un composant de type ECM non collagène ou ses dérivés étant intégré(s) dans celles-ci.

9. Implant ou support selon la revendication 8, **caractérisé en ce que** les composants de type ECM non collagènes contiennent des glycosaminoglycanes.

10. Implant ou support selon la revendication 8 ou 9, **caractérisé en ce que** le composant de type ECM non collagène contient du sulfate de chondroïtine de type A, C, D, ou E, du sulfate de dermatane, du sulfate de kératane, du sulfate d'héparane, de l'héparine, de l'acide hyaluronique et ses dérivés, seuls ou en mélange.

11. Implant ou support selon l'une quelconque des revendications 8-10, **caractérisé en ce que** le composant de type ECM non collagène contient des protéines de matrice non collagènes.

12. Implant ou support selon l'une quelconque des revendications 8-11, **caractérisé en ce que** le composant de type ECM non collagène contient, comme protéines de matrice non collagènes, de la fibronectine, de la décorine, du biglycan, de la laminine, du versican, seuls ou en mélange.

13. Implant ou support selon l'une quelconque des revendications 8-12, **caractérisé en ce que** le composant de collagène est constitué par un des collagènes I, II, III, V, IX, XI, ou leurs combinaisons.

14. Solution de revêtement contenant un composite de matrice ostéogène constitué par du collagène et d'au moins un composant de type ECM non collagène ou ses dérivés, **caractérisée en ce que** le composant de collagène est constitué par des fibrilles de collagène produites par fibrillogenèse, non réticulées et **en ce que** ledit au moins un composant de type ECM non collagène ou ses dérivés est/sont intégré(s) dans celles-ci.
